# EUROPÄISCHE PATENTSCHRIFT

(11) **EP 2 363 164 B1**
(45) Veröffentlichungstag und Bekanntmachung des Hinweises auf die Patenterteilung: **22.10.2014**
(21) Anmeldenummer: 11156796.2
(22) Anmeldetag: 03.03.2011
(51) Int. Cl.: A61M 21/00, A61H 23/02

(54) **Therapeutische Behandlungsliege**
Therapeutic treatment bed
Lit de soin thérapeutique

(30) Priorität: 04.03.2010 AT 12910 U
(43) Veröffentlichungstag der Anmeldung: 07.09.2011
(73) Patentinhaber: Kaltenreiner, Alfred, 4400 Steyr (AT)
(72) Erfinder: Kaltenreiner, Alfred, 4400 Steyr (AT)
(74) Vertreter: KLIMENT & HENHAPEL

(56) Entgegenhaltungen:
- EP-A1- 0 251 430
- EP-A2- 0 610 669
- WO-A-87/05497
- DE-A1- 4 117 694
- US-A- 2 821 191
- US-A1- 2008 129 094
- US-B2- 7 127 075

## Beschreibung

Die Erfindung betrifft eine therapeutische Behandlungsliege mit einer Auflagefläche für den Patienten, sowie zumindest einem, unterhalb der Auflagefläche angeordneten Schallkörper, der Schallwellen mit einer Frequenz unter 100 Hz erzeugt, gemäß dem Oberbegriff von Anspruch 1.

Behandlungsliegen dieser Art erzielen einen therapeutischen Effekt mithilfe der, durch die Niederfrequenz-Schwingungen erzeugten Tiefenentspannung des Patienten, sowie durch die eingebrachte Energie der Schallwellen, die etwa in Wärme übergeht, oder für eine Restrukturierung von Geweben, Bändern, Filamenten oder Muskeln sorgt. Gute Resultate konnten etwa bei Rücken-, Knie-, Hüft- und Kieferschmerzen, Durchfall und Verstopfung, Migräne und Regelbeschwerden, sowie bei chronischen Nebenhöhlen- und Blasenentzündungen nachgewiesen werden. Des Weiteren wirken Niederfrequenz-Schwingungen stresslösend und entkrampfend. Schallkörper der verwendeten Art, die Frequenzen unter 100 Hz erzeugen, sind auch als "Subwoofer" bekannt. Die so erzeugten Schwingungen übertragen sich direkt auf den Körper des Patienten und sind als Niederfrequenz-Schwingungen fühlbar. Durch die direkte Übertragung dieser Schwingungen auf den Körper anstatt einer akustischen Wahrnehmung unterliegen diese Schwingungen aber nicht der kognitiven Filterung.

EP 061066 9 A2 zeigt eine Vorrichtung zur therapeutischen Behandlung und vibratorischen Stimulation von Patienten, bei der in einem geschlossenen Gehäuse eines Hockers eine Schallmembran sehkrecht zur Sitzfläche angeordnet ist.

Dahingehend offenbart die US 2,821,191 A eine therapeutische Behandlungsliege mit einer Auflagefläche für den Patienten sowie zumindest einem, unterhalb der Auflagefläche in einem eigenen Resonanzkasten angeordneten Schallkörper, der Schallwellen erzeugt.

In herkömmlicher Art werden die Schallkörper unmittelbar an der Auflagefläche montiert, wodurch die Schwingungen direkt auf die Auflagefläche übertragen werden. Diese Ausführungsformen weisen allerdings den Nachteil auf, dass einerseits eine stark lokalisierte Abgabe der Schwingungen auf die Auflagefläche erfolgt, und zudem das Eigengewicht der Schallkörper die Schwingfähigkeit der Auflagefläche beeinträchtigt. Beides bewirkt ein ungleichförmiges Schwingungsmuster der Auflagefläche, wodurch der therapeutische Effekt beeinträchtigt wird.

Es ist daher das Ziel der Erfindung, die Auflagefläche gleichmäßiger in Schwingung zu versetzen, um ein optimales Schwingungsverhalten der Auflagefläche zu bewirken, und den therapeutischen Effekt dadurch zu verbessern.

Diese Ziele werden durch die Merkmale von Anspruch 1 erreicht. Anspruch 1 bezieht sich auf eine therapeutische Behandlungsliege mit einer Auflagefläche für den Patienten, sowie zumindest einem, unterhalb der Auflagefläche angeordneten Schallkörper, der Schallwellen mit einer Frequenz unter 100 Hz erzeugt, wobei erfindungsgemäß vorgesehen ist, dass unterhalb der Auflagefläche ein abgeschlossener Resonanzkasten vorgesehen ist, dessen obere Deckfläche Teil der Auflagefläche ist, und der zumindest eine Schallkörper im Innenraum des Resonanzkastens mit einer zur Auflagefläche im Wesentlichen senkrechten Schwingmembran angeordnet ist, wobei die Bodenfläche des Resonanzkörpers einen mittigen, zur Auflagefläche parallelen Abschnitt aufweist, sowie beidseits seitlich anschließende, zur Auflagefläche geneigte, schräge Abschnitte. Durch den erfindungsgemäß vorgesehenen Resonanzkasten wird die Schalleistung des Schallkörpers in eine gleichmäßige Schwingung der Begrenzungsflächen des Resonanzkastens umgesetzt, wobei die Deckfläche des Resonanzkastens Teil der Auflagefläche ist. Der Patient ruht somit im Wesentlichen auf der Deckfläche des Resonanzkastens, und wird somit unmittelbar dem gleichmäßigen Schwingungsbild des Resonanzkastens ausgesetzt.

Die Ausführung des Resonanzkastens kann auf unterschiedliche Weise erfolgen. Erfindungsgemäß wird vorgeschlagen, dass die Bodenfläche des Resonanzkörpers einen mittigen, zur Auflagefläche parallelen Abschnitt aufweist, sowie beidseits seitlich anschließende, zur Auflagefläche geneigte, schräge Abschnitte. Der mittige Abschnitt dient als Träger für den Schallkörper, wobei die seitlich anschließenden, schrägen Abschnitte den vom Schallkörper abgegebenen Schall in den Resonanzkasten zurück reflektieren, und eine Homogenisierung des Schallfeldes bewirken.

Vorzugsweise kann genau ein Schallkörper vorgesehen sein, der in der Mitte des Resonanzkastens angeordnet ist. Dadurch werden mitunter unerwünschte Interferenzeffekte vermieden.

Da der Resonanzkasten in seiner Gesamtheit schwingfähig sein soll, ist er entsprechend zu lagern oder aufzuhängen. Hierfür wird vorgeschlagen, dass eine zur Auflagefläche parallele, untere Trägerplatte für den Resonanzkasten vorgesehen ist, die einen Durchbruch aufweist, in dem der Resonanzkasten eingesetzt ist, wobei sich der Resonanzkasten mit seinen schrägen Abschnitten an den Randkanten des Durchbruchs abstützt. Für eine solche Ausführungsform hat sich in der Praxis eine gute Schwingfähigkeit des Resonanzkastens ergeben.

Des Weiteren wird vorgeschlagen, dass der Resonanzkörper mit losem Füllgut niedriger Dichte, vorzugsweise Wolle, befüllt ist. Ein solches Füllgut unterstützt die Homogenisierung des Schallfeldes im Inneren des Resonanzkastens.

Die Erfindung wird im Folgenden anhand eines Ausführungsbeispiels mithilfe der beiliegenden Figuren näher erläutert. Es zeigen hierbei die
Fig. 1 eine Ansicht einer Ausführungsform einer erfindungsgemäßen Behandlungsliege von oben gesehen, und die
Fig. 2 einen Querschnitt der erfindungsgemäßen Behandlungsliege gemäß Fig. 1.

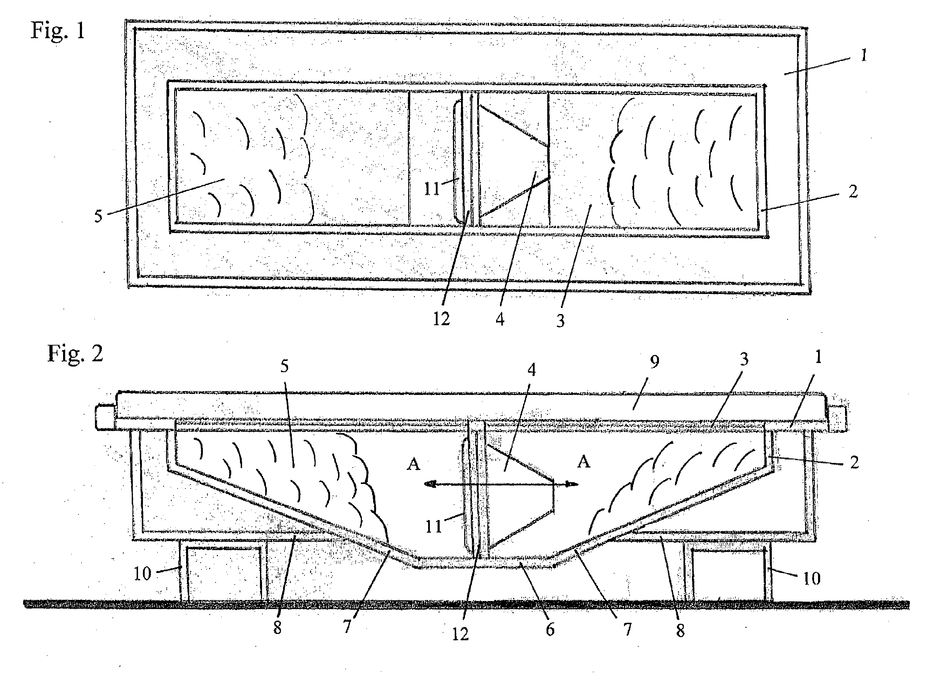

Die in Fig. 1 und Fig. 2 gezeigte Behandlungsliege gemäß der Erfindung weist eine Auflagefläche 1 auf, die in der Regel mit einer Liegematte 9 aus elastischem Material, etwa Schaumstoff und dergleichen, versehen sein wird. In der Behandlungsliege ist ein Resonanzkasten 2 integriert, dessen Deckfläche 3 mit der Auflagefläche 1 eben abschließt, sodass die Deckfläche 3 Teil der Auflagefläche 1 ist. Der Patient ruht somit größtenteils auf der Deckfläche 3. Eine mögliche Ausführungsform des Resonanzkastens 2 ist anhand der Fig. 2 ersichtlich. Er verfügt über eine Bodenfläche mit einem mittigen, zur Auflagefläche 1 parallelen Abschnitt 6, sowie beidseits seitlich anschließende, zur Auflagefläche 1 geneigte, schräge Abschnitte 7. Der Querschnitt des Resonanzkastens 2 ist somit im Wesentlichen trapezförmig ausgeführt. Des Weiteren ist eine zur Auflagefläche 1 parallele, untere Trägerplatte 8 für den Resonanzkasten 2 vorgesehen, die einen zentralen Durchbruch aufweisen kann, in dem der Resonanzkasten 2 eingesetzt ist, wobei sich der Resonanzkasten 2 mit seinen schrägen Abschnitten 7 an den Randkanten des Durchbruchs abstützt. Der Resonanzkasten 2 ist somit auf der Trägerplatte 8 schwingfähig gelagert. Die Trägerplatte 8 ist etwa auf Stützfüßen 10 stabil montiert. Der Resonanzkasten 2 ist vorzugsweise aus Holz gefertigt, generell werden natürliche Materialien, etwa Holz, zur Fertigung der gesamten Behandlungsliege bevorzugt.

Im Innenraum des Resonanzkastens 2 ist ein Schallkörper 4 mit einer zur Auflagefläche 1 im Wesentlichen senkrechten Schwingmembran 11 angeordnet. Im gezeigten Ausführungsbeispiel wird genau ein Schallkörper 4 verwendet, der in der Mitte des Resonanzkastens 2 angeordnet ist, und über eine Halterung 12, etwa eine Holzplatte, die im Resonanzkasten 2 eingesetzt ist, befestigt, vorzugsweise am mittigen Abschnitt 6 der Bodenfläche. Der Schallkörper 4 erzeugt Schall mit einer Frequenz von maximal 100 Hz, der entlang der Pfeilrichtungen A abgestrahlt wird. Die Ansteuerung des Schallkörpers 4 erfolgt mithilfe einer Steuereinheit (in den Fig. 1 und 2 nicht ersichtlich), die etwa an einer Außenwand der Behandlungsliege befestigt ist, und über Bedienelemente etwa zur Steuerung der Frequenz, der Schallleistung, oder der Impulsform des erzeugten Schallbildes des Schallkörpers 4 verfügt. Der Innenraum des Resonanzkastens 2 ist des Weiteren mit losem Füllgut 5 niedriger Dichte, vorzugsweise Wolle, befüllt.

Im Bedienzustand der erfindungsgemäßen Behandlungsliege strahlt der Schallkörper 4 akustische Schallwellen mit Frequenzen unterhalb von 100 Hz in den Innenraum des Resonanzkastens 2 in die Pfeilrichtungen A ab. Die seitlich anschließenden, schrägen Abschnitte 7 des Resonanzkastens 2 reflektieren den vom Schallkörper 4 abgegebenen Schall in den Innenraum des Resonanzkastens 2, und bewirken eine Homogenisierung des Schallfeldes, die durch das Füllgut 5 verstärkt werden kann. Durch den erfindungsgemäß vorgesehenen Resonanzkasten 2 wird somit die Schalleistung des Schallkörpers 4 in eine gleichmäßige Schwingung der Begrenzungsflächen des Resonanzkastens 2 umgesetzt. Dadurch wird die Auflagefläche 1, nämlich jener Teil, der durch die Deckfläche 3 des Resonanzkastens 2 gebildet wird, gleichmäßiger in Schwingung versetzt, und ein optimales Schwingungsverhalten der Auflagefläche 1 bewirkt, wodurch der therapeutische Effekt verbessert wird.

## Patentansprüche

1. Therapeutische Behandlungsliege mit einer Auflagefläche (1) für den Patienten, sowie zumindest einem, unterhalb der Auflagefläche (1) angeordneten Schallkörper (4), der Schallwellen mit einer Frequenz unter 100 Hz erzeugt, wobei unterhalb der Auflagefläche (1) ein abgeschlossener Resonanzkasten (2) vorgesehen ist, dessen obere Deckfläche (3) Teil der Auflagefläche (1) ist, und der zumindest eine Schallkörper (4) im Innenraum des Resonanzkastens (2) mit einer zur Auflagefläche (1) im Wesentlichen senkrechten Schwingmembran (11) angeordnet ist, wobei die Bodenfläche des Resonanzkörpers (2) einen mittigen, zur Auflagefläche (1) parallelen Abschnitt (6) aufweist, **dadurch gekennzeichnet, dass** die Bodenfläche des Resonanzkörpers (2) ferner beidseits seitlich anschließende, zur Auflagefläche (1) geneigte, schräge Abschnitte (7) aufweist.

2. Therapeutische Behandlungsliege nach Anspruch 1, **dadurch gekennzeichnet, dass** genau ein Schallkörper (4) vorgesehen ist, der in der Mitte des Resonanzkastens (2) angeordnet ist.

3. Therapeutische Behandlungsliege nach Anspruch 1, **dadurch gekennzeichnet, dass** eine zur Auflagefläche (1) parallele, untere Trägerplatte (8) für den Resonanzkasten (2) vorgesehen ist, die einen Durchbruch aufweist, in dem der Resonanzkasten (2) eingesetzt ist, wobei sich der Resonanzkasten (2) mit seinen schrägen Abschnitten (7) an den Randkanten des Durchbruchs abstützt.

4. Therapeutische Behandlungsliege nach einem der Ansprüche 1 bis 3, **dadurch gekennzeichnet, dass** der Resonanzkörper (2) mit losem Füllgut niedriger Dichte, vorzugsweise Wolle, befüllt ist.

## Claims

1. A therapeutic treatment bed, comprising a contact surface (1) for a patient, and at least one sonic body (4) which is arranged beneath the contact surface (1) and generates sound-waves with a frequency of less than 100 Hz, wherein a sealed resonance box (2) is provided beneath the contact surface (1) whose upper cover surface (3) is part of the contact surface (1), and the at least one sonic body (4) is arranged in the interior space of the resonance box (2) with an oscillating diaphragm (11) which is substantially perpendicular to the contact surface (1), wherein the bottom surface of the resonance body (2) comprises a central section (6) which is parallel to the contact surface (1), **characterized in that** the bottom surface of the resonance body (2) further comprises oblique sections (7) which are inclined to the contact surface (1) and are laterally adjacent on both sides.

2. A therapeutic treatment bed according to claim 1, **characterized in that** precisely one sonic body (4) is provided which is arranged in the centre of the resonance box (2).

3. A therapeutic treatment bed according to claim 1, **characterized in that** a bottom support plate (8) which is parallel to the contact surface (1) is provided for the resonance box (2), which support plate comprises a breakthrough in which the resonance box (2) is inserted, wherein the resonance box (2) rests with its oblique sections (7) on the boundary edges of the breakthrough.

4. A therapeutic treatment bed according to one of the claims 1 to 3, **characterized in that** the resonance body (2) is filled with loose filling material of low density, preferably wool.

## Revendications

1. Fauteuil de traitement thérapeutique avec une surface de couchage (1) pour le patient ainsi qu'au moins corps sonore (4) situé en-dessous de la surface de couchage (1), lequel génère des ondes sonores à une fréquence inférieure à 100 Hz, sachant que sous la surface de couchage (1), est prévu un caisson de résonnance fermé (2) dont la plaque supérieure (3) constitue une partie de la surface de couchage (1) et que le corps sonore (4) au moins au nombre de un, est disposé à l'intérieur du caisson de résonnance (2) avec une membrane oscillante (11) essentiellement perpendiculaire à la surface de couchage (1), sachant que le fond du corps de résonnance (2) présente une section (6) médiane, parallèle à la surface de couchage (1), **caractérisé en ce que** le fond du corps de résonnance (2) présente également des deux côtés, des sections (7) latérales, inclinées et obliques par rapport à la surface de couchage (1).

2. Fauteuil de traitement thérapeutique selon la revendication 1, **caractérisé en ce que** précisément un corps sonore (4) est prévu, lequel est disposé au milieu du caisson de résonnance (2).

3. Fauteuil de traitement thérapeutique selon la revendication 1, **caractérisé en ce qu'**une plaque support (8) inférieure, parallèle à la surface de couchage (1) est prévue pour le caisson de résonnance (2) et présente une ouverture dans laquelle le caisson de résonnance (2) est placé, sachant que le caisson de résonnance (2) s'appuie sur les arêtes du bord de l'ouverture avec des sections obliques (7).

4. Fauteuil de traitement thérapeutique selon l'une des revendications 1 à 3, **caractérisé en ce que** le corps de résonnance (2) est rempli d'une matière en vrac de faible densité, de préférence de la laine.
